# EUROPEAN PATENT APPLICATION

(11) **EP 2 783 680 A1**
(43) Date of publication of application: **01.10.2014**
(21) Application number: 14161242.4
(22) Date of filing: 24.03.2014
(51) Int. Cl.: A61K 9/20, A61K 31/155, A61K 31/403

(54) **Controlled-release formulations comprising metformin and gliclazide**

(30) Priority: 25.03.2013 TR 201303559
(71) Applicant: Sanovel Ilac Sanayi ve Ticaret A.S., 34460 Istanbul (TR)
(72) Inventor: Türkyilmaz, Ali, 34460 Istanbul (TR); Pehlivan Akalin, Nur, 34460 Istanbul (TR); Sucuoglu, Esra, 34460 Istanbul (TR); Okyar, M Isin Ruiye, 34460 Istanbul (TR)
(74) Representative: Sevinç, Erkan

(57) **Abstract**

The present invention relates to a formulation comprising metformin or a pharmaceutically acceptable salt thereof and gliclazide or a pharmaceutically acceptable salt thereof. The present invention more particularly relates to a controlled-release formulation of metformin and gliclazide.

## Description

### Field of Invention

The present invention relates to a formulation comprising metformin or a pharmaceutically acceptable salt thereof and gliclazide or a pharmaceutically acceptable salt thereof. The present invention more particularly relates to a controlled-release formulation of metformin and gliclazide.

### Background of Invention

Diabetes mellitus is a metabolic anomaly of the glucose metabolism occurring, *inter alia*, due to inadequate insulin secretion and decreased sensitivity of insulin target cells and is basically characterized by recognizable hyperglycemia. In the persistent hyperglycemia, severe harmful complications such as retinopathy, nephropathy and neuropathy occur as a result of vascular lesions in various organs and nerves.

Many anti-diabetic drugs, e.g. sulfonylureas, have been administered orally in the treatment of diabetics so far.

Sulfonylurea is a name defining a group of orally administered anti-diabetic drugs used in the treatment of type II diabetes. Diabetes is a disorder of the carbohydrate metabolism, in which insulin secretion is diminished, or which occurs due to a varying secretion or decreased insulin activity or a combination of both factors. It is believed that sulfonylureas stimulate the insulin secretion from pancreatic islet cells in the mediation of receptors reported to be adenosine 5'-triphosphate-sensitive potassium channels. For this reason, sulfonylureas basically increase the endogenous insulin secretion so that an efficient control is provided in the blood sugar levels of the diabetics, particularly of the type II diabetics whose blood sugar levels cannot be controlled via diets.

Sulfonylureas are considered to be divided into two categories: first generation agents, e.g. tolbutamide, chlorpropamide, tolazamide, acetohexamide; and second generation agents, e.g. glyburide (glibenclamide), glipizide, gliclazide.

Gliclazide, N-(4-methylbenzenesulfonyl)-N'-(3-azabicyclo[3.3.0]-oct-3-yl)urea), one type of second generation sulfonylureas, is an active agent which has antidiabetic properties at typical doses administered to the humans (the chemical structure thereof is illustrated below in Formula I).

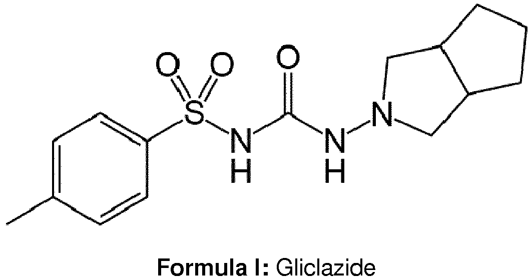

Gliclazide has been administered in the form of 30 mg and 80 mg tablets. A regular administration of gliclazide is twice a day, but it may be altered to 1 to 4 tablets a day, as required by the severity of the diabetes case. Additionally, 30 mg and 60 mg modified-release dosage forms are available at the markets.

The formulation of gliclazide as an active ingredient was first disclosed in the patent document US 3,501,495 (SCIENCE UNION ET CIE. SOC.) on 10.02.1996, disclosing the formula N-(4-methylbenzenesulphonyl)-N'-(3-azabicyclo[3.3.0]-oct-3-yl)urea) having hypoglycemic properties and being used orally in the treatment of diabetes.

Metformin hydrochloride, with the chemical name 1,1-dimethylbiguanide hydrochloride, has the following chemical structure of Formula II.

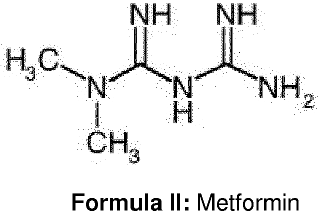

Metformin is an oral antidiabetics having a biguanide structure and was first disclosed in the patent US 3,174,901. It is used singly or in combination with sulfonylureas, alpha-glucosidase inhibitors, or with insulin in the treatment of diabetes. It is freely dissolved in water.

Various patent applications have been filed in relation to metformin and gliclazide in the prior art. Although that these active agents are used singly in the treatment of type II diabetes, the concurrent use of both agents was observed to be more efficient than their individual uses. Metformin reduces the hepatic glucose production and develops insulin sensitivity with peripheral glucose uptake. Metformin is taken twice a day, whereas gliclazide is taken 2 to 3 times per day. The number of doses daily taken is reduced by controlled-release tablets.

The patents US 6,099,862 and US 6,284,275 disclose a combination comprising a biguanide and a sulfonylurea to provide a concurrent controlled-release. Biguanide is preferably metformin or buformin, whereas the sulfonylurea is preferably glipizide. The core which comprises these two active agents is coated by a semi-permeable coating, wherein the controlled release is ensured by means of a passageway extending up to the core. However, special apparatuses are required to produce the passageway extending from the coating to the core and therefore the production cost of this method is high.

Tablets are known from the prior art, which comprise a biguanide and a sulfonylurea and have concurrent controlled-release layers. The concurrent controlled-release layers, in turn, ensure a sustained blood sugar balance. However, there is a need for simple and inexpensive production methods for such embodiments.

The selection and ratio of the excipients, which are to provide a suitable release profile, and at the same time a controlled-release of the layers are quite critical for such drug embodiments.

Considering the aforesaid problems, it is obvious that a novelty is needed for both providing production convenience in the art of "once a day" dose formulations comprising metformin hydrochloride and gliclazide, and ensuring convenient release profiles for the same.

### Object and Brief Description of Invention

The present invention provides a bilayer tablet formulation comprising metformin and gliclazide, eliminating all of the aforesaid problems and brining additional advantages to the relevant prior art.

Accordingly, the main object of the present invention is to obtain a bilayer tablet formulation, which is stable and has a desired level of dissolution rate.

Another object of the present invention is to provide a stable formulation of metformin and gliclazide for oral use once or twice a day by virtue of a simple and low-cost production method.

A further object of the present invention is to maintain the effect of the formulation for 12-24 hours.

A bilayer tablet formulation having a controlled-release layer comprising metformin or a pharmaceutically acceptable salt thereof, and a controlled-release layer comprising gliclazide or a pharmaceutically acceptable salt thereof has been developed to carry out all objects, referred to above and to be described in the following detailed description.

### Detailed Description of Invention

"Modified-release dosage forms" is defined by the USP (United States Pharmacopeia) as dosage forms of which the drug-release characteristics of time course are chosen to accomplish therapeutic or convenience objectives not offered by conventional dosage forms. Extended-release dosage form provides a two-fold decrease in the dosing frequency and an increase in the patient compliance and therapeutic performance. According to the USP, the terms "controlled-release", "extended-release", and "sustained-release" may be used in place of the term "extended-release". Accordingly, the terms "modified-release", "controlled-release", "extended-release", and "sustained-release" may be used interchangeably.

In recent years, there has been a substantial increase in the development and use of extended-release formulations. These formulations are designed to release the respective active agent in a slower rate following administration as compared to a case in which the drug agent is used alone or in an inert formulation. Controlled-release formulations may provide improved therapeutic effects, a decreased incidence of side effects, and simplified dosing regimes. A controlled-release tablet generally releases a drug agent included therein in a long time period, typically in a time longer than 3 hours. On the other hand, a drug agent included in a controlled-release formulation is released in a time period shorter than 30 hours. These known controlled-release formulations make use of various mechanism to control the release of an active agent included therein. The selected specific mechanism largely depends on the therapeutic targets of the formulation. Some representative mechanisms used in controlled-release formulations comprise dissolution, diffusion, swelling, osmotic pressure, complex formation, ion exchange, and tablet erosion.

A controlled-release form has many advantages over an immediate-release form. It eliminates the toxic side effects or eliminates the effect reduction and the total drug amount given to provide the same blood levels over a determined time period is less. Additionally, since it eliminates the need for frequent drug administration, it reduces the dependency of the treatment's efficiency on the patient and may allow for chronotherapeutic approaches.

In this context, polymers can be used to form a matrix in which the active agent is dispersed. The release rate of the active agent depends on the properties of the polymer used. Cellulose derivatives used are particularly hydroxypropyl methylcellulose (HPMC) and hydroxypropyl cellulose. The properties of a polymer when it makes contact with an aqueous medium and particularly a physiological medium can be used for controlling the release rate of an active agent from a controlled-release formulation. For instance, polymers like HPMC frequently develop a gel-like outer layer that is dissolved or worn away and thus control the permeation of water into the interior of a controlled-release formulation. It is known that the release of an active agent from such a controlled-release formulation is controlled by a combination of many factors, including the properties of the active agent, the diffusion properties of the active agent or of the solution in which it is just dissolved, the penetration rate of water into the interior of the polymer matrix, swelling features of the polymer matrix, and the dissolution rate of the gel layer of the outer polymer.

According to the present invention, hydroxypropyl methylcellulose (HPMC) which is a high viscous polymer with a viscosity above 1000 cP is used as a release rate-controlling polymer to provide the concurrent controlled-release. Keeping the amounts of the active agents in both layers above the amounts of the high-viscous HPMC in the same layers made it surprisingly possible to provide a concurrent controlled-release in both layers. HPMC K100 MCR (100000 cP) or HPMC E4M (4000 cP) is used as the high-viscous HPMC.

According to a preferred embodiment, the novelty according to the present invention is realized by setting the amounts of the active agents in the controlled-release layers to the amounts of the high-viscous HPMC in the same layers, respectively, in between 1:2 and 10:1, preferably 1:1 and 5:1. While higher amounts can give rise to negative effects on the mechanical resistance of the formulation, lower amounts may worsen the controlled-release capability of said layers.

According to another embodiment of the present invention, it was further observed that setting the percentage of the total amount of the binders present in both layers to the total tablet weight between 3.0% and 15.0% was useful in making the matrix together with the release rate controlling polymer and in providing the controlled-release of the layers. Thus, a stable once-a-day or twice-a-day oral formulation could be obtained which comprises metformin and gliclazide. A low-viscosity hydroxypropyl cellulose or carboxymethylcellulose sodium having a viscosity below 150 cP was used as a binder in both layers. HPMC E5-LV (5 cP) or HPMC K100LV (100 cP) is used as the low-viscous HPMC.

As used herein, the term "viscosity" means that the property of resistance to flow in a fluid or resistance of a fluid to a change in shape, or movement of neighboring portions relative to one another. Therefore, optimization of viscosity grade provides the desired dissolution properties.

Furthermore, the stable formulation of metformin and gliclazide was obtained by using a wet granulation method in both layers and therefore a simple and low-cost production method was employed.

The pharmaceutical formulations according to the present invention may also comprise one or more pharmaceutically acceptable excipient(s). Pharmaceutically acceptable excipients include, but are not limited to fillers, lubricants and glidants.

Suitable fillers for use in a formulation according to the present invention include, but are not limited to sugars, mannitol, sorbitol, sucrose, inorganic salts, calcium salts, polysaccharides, dextrose, dicalcium phosphate, sodium chloride, dextrates, lactitol, maltodextrin, sucrose-maltodextrin mixtures, xylitol, trehalose, heavy magnesium carbonate, microcrystalline cellulose, calcium hydrogen phosphate dihydrate, or the mixtures thereof. In a tablet formulation according to the present invention, the percentage of the total amount of fillers in both layers to the total tablet weight is between 1.0% and 30.0%, preferably 5.0% and 25.0%.

Suitable lubricants for use in a formulation according to the present invention include, but are not limited to magnesium stearate, sodium stearyl fumarate, polyethylene glycol, stearic acid, metal stearates, boric acid, sodium chloride benzoate and acetate, sodium lauryl sulfate, magnesium lauryl sulfate, or the mixtures thereof. In a tablet formulation according to the present invention, the percentage of the total amount of lubricants in both layers to the total tablet weight is between 0.01 % and 10.0%.

Suitable glidants for use in a formulation according to the present invention include, but are not limited to silicon dioxide, magnesium trisilicate, starch, talk, colloidal silicon dioxide, silicone hydrogel, or the mixtures thereof. In a tablet formulation according to the present invention, the percentage of the total amount of glidants in both layers to the total tablet weight is between 0.01% and 10.0%.

Thus, the present invention relates to obtaining a stable and sustained-release bilayer tablet formulation comprising gliclazide or a pharmaceutically acceptable salt thereof, metformin or a pharmaceutically acceptable salt thereof, a high-viscosity HPMC as a matrix builder, and a low-viscosity HPMC or carboxymethylcellulose sodium as a binder, which when stored under accelerated conditions such as 25°C and 60% relative humidity or 40°C and 75% relative humidity shows an impurity less than 0.4%.

The present invention is for use in the prevention or in the treatment of diabetes and particularly type II diabetes, noninsulin-dependent diabetes mellitus, in the mammalians and particularly in the humans.

The present invention will be further disclosed in the following examples. These examples do not restrict the present invention and the present invention must be assessed under the light of the foregoing detailed description.

### Example

### Example 1:

| ***1^{st} Layer:*** | **Amount (%)** |
|---|---|
| Gliclazide | 15.0 - 22.0 |
| Hydroxypropyl methylcellulose (E4M) | 5.0 - 10.0 |
| Calcium hydrogen phosphate dihydrate | 4.0 - 8.5 |
| Hydroxypropyl methylcellulose (K100LV) | 5.0 - 10.0 |
| Mannitol | 50.0 - 60.0 |
| Colloidal silicon dioxide | 0.05 - 0.5 |
| Magnesium stearate | 0,25 - 1.0 |

| ***2^{nd} Layer:*** | **Amount (%)** |
|---|---|
| Metformin hydrochloride | 40.0 - 55.0 |
| Hydroxypropyl methylcellulose (E5-LV) | 0.5 - 1.5 |
| Hydroxypropyl methylcellulose (K100M CR) | 30.0 - 40.0 |
| Carboxymethylcellulose sodium | 3.0 - 6.0 |
| Microcrystalline cellulose | 9.0 - 12.0 |
| Magnesium stearate | 0,25 - 1.0 |

| | |
|---|---|
| **The percent amounts given were calculated separately for each layer.* **1^{st} Layer:** → Gliclazide, calcium hydrogen phosphate dihydrate, mannitol, and some portion of hydroxypropyl methylcellulose (E4M) are mixed in a granulator. Water is sprayed onto the resulting mixture and is thus granulated. → The wet granules prepared are directly transferred to a fluidized bed dryer. The granules are dried in the fluidized bed dryer until the humidity of the granules are reduced to below 1% maximum, and dried granules are sieved and passed to the mixer. → The remaining part of hydroxypropyl methylcellulose (E4M), the entirety of hydroxypropyl methylcellulose (K100LV), and colloidal silicon dioxide are added to the sieved dry granules and the resulting mixture is stirred. Then, magnesium stearate is added thereto and it is stirred for a while. **2^{nd} Layer:** → Weight amounts of metformin hydrochloride and carboxymethylcellulose sodium are mixed and wet granulation is carried out using pure water. The mixture is passed through a wet granulator and taken to an oven tray. → It is dried in an oven to a desired humidity value and is sieved into an IBC*. Hydroxypropyl methylcellulose having a viscosity of 5 cP (E5 -LV), hydroxypropyl methylcellulose having a viscosity of 100000 cP (K100 MCR) and microcrystalline cellulose are taken to an IBC and stirred therein. Then, magnesium stearate is added thereto and stirred for a while. → The mixtures prepared are compressed into two layers and combined in a tablet. *IBC = Intermediate Bulk Container. | |

### Example 2:

| ***1^{st} Layer:*** | **Amount (%)** |
|---|---|
| Gliclazide | 15.0 - 22.0 |
| Hydroxypropyl methylcellulose (E4M) | 9.0 - 12.0 |
| Calcium hydrogen phosphate dihydrate | 40.0 - 50.5 |
| Hydroxypropyl methylcellulose (K100LV) | 9.0 - 14.0 |
| Mannitol | 10.0 - 15.0 |
| Colloidal silicon dioxide | 0.05 - 0.5 |
| Magnesium stearate | 0.25 - 1.0 |

| ***2^{nd} Layer:*** | **Amount (%)** |
|---|---|
| Metformin hydrochloride | 40.0 - 55.0 |
| Hydroxypropyl methylcellulose (E5-LV) | 0.5 - 1.5 |
| Hydroxypropyl methylcellulose (K100M CR) | 30.0 - 40.0 |
| Carboxymethylcellulose sodium | 3.0 - 6.0 |
| Microcrystalline cellulose | 9.0 - 12.0 |
| Magnesium stearate | 0.25 - 1.0 |

| | |
|---|---|
| **The percent amounts given were calculated separately for each layer.* **1^{st} Layer:** → Gliclazide, calcium hydrogen phosphate dihydrate, mannitol, and some portion of hydroxypropyl methylcellulose having a viscosity of 4000 cP (E4M) are mixed in a granulator. Water is sprayed onto the resulting mixture and is thus granulated. → The wet granules prepared are directly transferred to a fluidized bed dryer. The granules are dried in the fluidized bed dryer until the humidity of the granules are reduced to below 1% maximum, and dried granules are sieved and passed to the mixer. → The remaining part of hydroxypropyl methylcellulose (E4M), the entirety of hydroxypropyl methylcellulose having a viscosity of 100 cP (K100LV), and colloidal silicon dioxide are added to the sieved dry granules and the resulting mixture is stirred. Then, magnesium stearate is added thereto and it is stirred for a while. **2^{nd} Layer:** → Weight amounts of metformin hydrochloride and carboxymethylcellulose sodium are mixed and wet granulation is carried out using pure water. The mixture is passed through a wet granulator and taken to an oven tray. → It is dried in an oven to a desired humidity value and is sieved into an IBC. Hydroxypropyl methylcellulose having a viscosity of 5 cP (E5 -LV), hydroxypropyl methylcellulose having a viscosity of 100000 cP (K100 MCR) and microcrystalline cellulose are taken to an IBC and stirred therein. → Then, magnesium stearate is added thereto and stirred for a while. → The mixtures prepared are compressed into two layers and combined in a tablet. | |

### Example 3:

| ***1^{st} Layer:*** | **Amount (%)** |
|---|---|
| Gliclazide | 15.0 - 22.0 |
| Hydroxypropyl methylcellulose (E4M) | 5.0 - 10.0 |
| Calcium hydrogen phosphate dihydrate | 4.0 - 8.5 |
| Hydroxypropyl methylcellulose (K100LV) | 5.0 - 10.0 |
| Mannitol | 50.0 - 60.0 |
| Colloidal silicon dioxide | 0.05 - 0.5 |
| Magnesium stearate | 0.25 - 1.0 |

| ***2^{nd} Layer:*** | **Amount (%)** |
|---|---|
| Metformin hydrochloride | 65.0 - 79.0 |
| Hydroxypropyl methylcellulose (K100 LV) | 0.5 - 3.0 |
| Hydroxypropyl methylcellulose (K100M CR) | 18.0 - 28.0 |
| Carboxymethylcellulose sodium | 3.0 - 7.0 |
| Magnesium stearate | 0.25 - 1.0 |

| | |
|---|---|
| **The percent amounts given were calculated separately for each layer.* **1^{st} Layer:** → Gliclazide, calcium hydrogen phosphate dihydrate, mannitol, and some portion of hydroxypropyl methylcellulose (E4M) are mixed in a granulator. Water is sprayed onto the resulting mixture and is thus granulated. → The wet granules prepared are directly transferred to a fluidized bed dryer. The granules are dried in the fluidized bed dryer until the humidity of the granules are reduced to below 1% maximum, and dried granules are sieved and passed to the mixer. → The remaining part of hydroxypropyl methylcellulose (E4M), the entirety of hydroxypropyl methylcellulose (K100LV), and colloidal silicon dioxide are added to the sieved dry granules and the resulting mixture is stirred. → Then, magnesium stearate is added thereto and it is stirred for a while. **2^{nd} Layer:** → A weighed amount of metformin hydrochloride is introduced into a granulator and mixed for 10 minutes. Then, carboxymethylcellulose sodium and hydroxypropyl methylcellulose having a viscosity of 100 cP (K100 LV) are added and mixed for 5 minutes. Water is sprayed onto the resulting mixture and is thus granulated. → The granules are sieved and are added to the fluidized bed dryer and dried. The dried granules are passed through a sieve and granulated. → Hydroxypropyl methylcellulose having a viscosity of 100000 cP (K100 MCR) is added to the sieved dry granules and stirred for around 15 minutes. Then, magnesium stearate is added thereto and it is stirred for around 3 minutes. | |

The mixtures prepared are compressed into two layers and combined in a tablet.

### Example 4:

| ***1^{st} Layer:*** | **Amount (%)** |
|---|---|
| Gliclazide | 15.0 - 22.0 |
| Hydroxypropyl methylcellulose (E4M) | 9.0 - 12.0 |
| Calcium hydrogen phosphate dihydrate | 40.0 - 50.5 |
| Hydroxypropyl methylcellulose (K100LV) | 9.0 - 14.0 |
| Mannitol | 10.0 - 15.0 |
| Colloidal silicon dioxide | 0.05 - 0.5 |
| Magnesium stearate | 0,25 - 1.0 |

| ***2^{nd} Layer:*** | **Amount (%)** |
|---|---|
| Metformin hydrochloride | 65.0 - 79.0 |
| Hydroxypropyl methylcellulose (K100 LV) | 0.5 - 3.0 |
| Hydroxypropyl methylcellulose (K100M CR) | 18.0 - 28.0 |
| Carboxymethylcellulose sodium | 3.0 - 7.0 |
| Magnesium stearate | 0,25 - 1.0 |

| | |
|---|---|
| **The percent amounts given were calculated separately for each layer.* **1^{st} Layer:** → Gliclazide, calcium hydrogen phosphate dihydrate, mannitol, and some portion of hydroxypropyl methylcellulose (E4M) are mixed in a granulator. Water is sprayed onto the resulting mixture and is thus granulated. → The wet granules prepared are directly transferred to a fluidized bed dryer. The granules are dried in the fluidized bed dryer until the humidity of the granules are reduced to below 1% maximum, and dried granules are sieved and passed to the mixer. → The remaining part of hydroxypropyl methylcellulose (E4M), the entirety of hydroxypropyl methylcellulose (K100LV), and colloidal silicon dioxide are added to the sieved dry granules and the resulting mixture is stirred. Then, magnesium stearate is added thereto and it is stirred for a while. **2^{nd} Layer:** → A weighed amount of metformin hydrochloride is introduced into a granulator and mixed for 10 minutes. Then, carboxymethylcellulose sodium and hydroxypropyl methylcellulose having a viscosity of 100 cP (K100 LV) are added and mixed for 5 minutes. Water is sprayed onto the resulting mixture and is thus granulated. → The granules are sieved and are added to the fluidized bed dryer and dried. The dried granules are passed through a sieve and granulated. → Hydroxypropyl methylcellulose having a viscosity of 100000 cP (K100 MCR) is added to the sieved dry granules and stirred for around 15 minutes. Then, magnesium stearate is added thereto and it is stirred for around 3 minutes. → The mixtures prepared are compressed into two layers and combined in a tablet. | |

## Claims

1. A bilayer tablet formulation having a controlled-release layer comprising metformin or a pharmaceutically acceptable salt thereof and a second controlled-release layer comprising gliclazide or a pharmaceutically acceptable salt thereof, wherein both layers comprise a high-viscosity HPMC (hydroxypropyl methylcellulose) having a viscosity above 1000 cP and wherein the amounts of the active agents present in both layers are more than the amount of high-viscosity HPMC present in both layers, respectively.

2. The tablet formulation according to claim 1, wherein the ratio of the amount of the active agents present in two layers to the amount of high-viscosity HPMC in the same layers is between 1:2 and 10:1, respectively.

3. The tablet formulation according to claim 2, wherein the ratio of the amount of the active agents present in two layers to the amount of high-viscosity HPMC in the same layers is between 1:1 and 5:1, respectively.

4. The tablet formulation according to claims 1 to 3, wherein the percentage of the total amount of binders in both layers to the total tablet weight is between 3.0% and 15.0%.

5. The tablet formulation according to claim 4, wherein both layers comprise carboxymethylcellulose sodium or a low-viscosity HPMC with a viscosity below 150 cP as a binder.

6. The tablet formulation according to any of the preceding claims, further comprising at least one pharmaceutically-acceptable excipient selected from a group consisting of fillers, lubricants and glidants.

7. The tablet formulation according to claim 6, wherein the filler is selected from a group comprising sugars, mannitol, sorbitol, sucrose, inorganic salts, calcium salts, polysaccharides, dextrose, dicalcium phosphate, sodium chloride, dextrates, lactitol, maltodextrin, sucrose-maltodextrin mixtures, xylitol, trehalose, heavy magnesium carbonate, microcrystalline cellulose, calcium hydrogen phosphate dihydrate, and the mixtures thereof.

8. The tablet formulation according to claim 7, wherein the percentage of the total amount of fillers in both layers to the total tablet weight is between 1.0% and 30.0%, preferably 5.0% and 25.0%.

9. The tablet formulation according to claim 6, wherein the lubricant is selected from a group comprising magnesium stearate, sodium stearyl fumarate, polyethylene glycol, stearic acid, metal stearates, boric acid, sodium chloride benzoate and acetate, sodium lauryl sulfate, magnesium lauryl sulfate, and the mixtures thereof.

10. The tablet formulation according to claim 6, wherein the glidant is selected from a group comprising silicon dioxide, magnesium trisilicate, starch, talk, colloidal silicon dioxide, silicone hydrogel, and the mixtures thereof.

11. The pharmaceutical formulation according to any of the preceding claims for use in the prevention or treatment of diabetes mellitus in the mammalians and particularly in the humans.
